# EUROPEAN PATENT APPLICATION

(11) **EP 3 845 519 A1**
(43) Date of publication of application: **07.07.2021**
(21) Application number: 19220141.6
(22) Date of filing: 30.12.2019
(51) Int. Cl.: C07C 227/18, C07C 229/08, C08G 59/00, C08G 69/00, C08G 71/00, C09J 177/00, C09J 185/00

(54) **ESTERS OF AMINO CARBOXYLIC ACIDS AND A PROCESS TO PREPARE THEM**

(71) Applicant: Nouryon Chemicals International B.V., 6824 BM Arnhem (NL)
(72) Inventor: SMOLKO-SCHVARZMAYR, Natalija, 444 85 Stenungsund (SE); WANG, Ruifie, 444 85 Stenungsund (SE); WETZEL, Alexander, Michael, 444 85 Stenungsund (SE); SCHWARZMAYR, Louis, 444 85 Stenungsund (SE); LJUNGDAHL, Göran, Thomas, 444 85 Stenungsund (SE); KOLMAN, Krzysztof, 444 85 Stenungsund (SE)
(74) Representative: LKGlobal UK Ltd.

(57) **Abstract**

The present invention relates to a process to prepare amine-functional ester 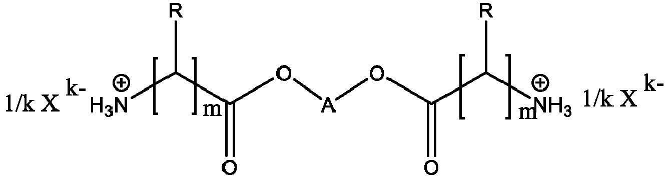 comprising the steps of reacting a polyol with an aminocarboxylic acid or its cyclic amide in the presence of a Bronsted-Lowry acid at a temperature of between 60 and 200 degrees C wherein the total molar amount of aminocarboxylic acid or its cyclic amide to the molar amount of the polyol is between n:0.8 and n:1.5 wherein n is the total number of hydroxyl groups on the polyol and wherein the Bronsted-Lowry acid is not added to the reaction mixture until at least 50% of the total of the polyol and the aminocarboxylic acid or its cyclic amide are dosed, wherein A is a carbon chain with 2 to 36 carbon atoms that can be aliphatic (linear or branched, saturated or unsaturated) or aromatic, or CH₂CH(OH)CH₂, CH₂C(CH₂OH)₂CH₂, CH₂C(CH₂OH)(CH₃)CH₂, CH₂C(CH₂OH)(CH₂CH₃)CH₂, p-tetrahydrofuran, erythritol or an ester of di- or tricarboxylic acids with ethylene or propylene glycol, and wherein A can optionally be alkoxylated or reacted with hydroxy carboxylic acids, m is an integer of 1-8 in formula II and 3-8 in formula III, each R independently is hydrogen or a C1-C4 alkyl group, or CH₂CH₂COOH, or CH₂COOH, or (CH₂)₄NH₂, k is a value of 1 to 3, and X is an anion derivable from deprotonating the Bronsted-Lowry acid. The invention also relates to the amine-functional esters obtained by the process as well as their use.

## Description

The present invention relates to a process to prepare amine-functional esters of amino carboxylic acids and polyols and to the amine-functional esters obtainable by such process.

In a number of documents the reaction of a monoalcohol with an amino carboxylic acid is disclosed.

For example, a process to prepare esters of aminocarboxylic acids is disclosed in "Esters of 6-aminohexanoic acid as skin permeation enhancers: The effect of branching in the alkanol moiety", A Habralek et al, Journal of Pharmaceutical Sciences, Vol. 94, 1494-1499, (2005). The process disclosed in this document involves in a first step a conversion of the amino carboxylic acid to an amino acyl chloride that next reacts with an alkanol to give an ammonium functional ester that next is deprotonated to give the aminocarboxylic acid ester.

JP S49-082624 discloses a process to prepare esters of aminocarboxylic acids in which an aminocarboxylic acid or a cyclic amide thereof is first reacted with a mineral acid catalyst which will convert this aminocarboxylic acid or cyclic amide into its aminocarboxylic acid salt, and next this aminocarboxylic acid salt is esterified with an alkanol to give the ester of the alkanol and the aminocarboxylic acid. This process is hence an esterification (or condensation) reaction, in embodiments wherein the cyclic amide precursor is employed, preceded by a de-esterification (or hydrolysis) reaction. The process is done at a temperature of 150 deg C and the alkanol reactant is 20% overdosed on cyclic amide molar amount.

J Klimentova, et al, in "Transkarbams with terminal branching as transdermal permeation enhancers", Bioorganic & Medicinal Chemistry Letter 18 (2008), 1712-1715 disclose also an esterification reaction of the salt of an aminocarboxylic acid with an alkanol in the presence of thionylchloride. The molar ratio of aminocarboxylic acid salt to alkanol in this document is 6.16: 5.54 which corresponds with 1.11:1.00.

US 4,216,227 discloses a process to prepare omega amino acid esters by reacting omega amino acid salts with an excess of alkanol in the presence of dry HCI gas. The details of the process are not disclosed, other than that a temperature of 150 deg C is employed for 3 hours, and neither is the use of cyclic amide instead of amino acid salt disclosed.

EP 847987 also discloses a process to prepare esters of aminocarboxylic esters. The process disclosed in EP 987 involves reacting an aminocarboxylic acid or a cyclic amide thereof with an alkanol in the presence of a divalent or trivalent inorganic acid, wherein the alkanol is dosed in high molar excess relative to the amount of the aminocarboxylic acid and later distilled off. The process is said to be performed under reflux conditions for several hours.

US 3,211,781, like EP 847987, discloses a process to prepare esters of amino carboxylic acids wherein a cyclic amide of an amino carboxylic acid is reacted with an alkanol in the presence of a Bronsted-Lowry acid under reflux conditions. Also, in this document the alkanol is dosed in a high molar excess compared to the molar amount of cyclic amide.

Lele, Gore and Kulkarni in Direct Esterification of Poly)ethylene glycol) with amino acid hdyrochlorides, Synthetic Communications, 29:10, 1727-1739 (1999) disclose a process of reacting polyethyleneglycol PEG 6000 with an amino acid in the HCI form in the presence of dicyclohexyl carbodiimide

US 3,939,200 discloses a process of reacting an amino acid in its salt form with a diol wherein the diol is an alkane or cycloalkane diol. The amino acids are first converted to their, preferably HCI, salt. It is indicated that when a cyclic amide is employed it will have to be first hydrolyzed to ring open the structure. In the Examples solvents such as trichloropropane or toluene are used in a quite high amount. Halogenated alkanes and aromatics are undesirable solvents as they have HSE issues, such as suspected carcinogenity.

It has been found that a process to prepare amine-functional esters of aminocarboxylic acids and polyols can be performed by dosing the polyol and (the cyclic amide of) aminocarboxylic acid in the right molar ratios of about n moles of aminocarboxylic on each mole polyol containing n hydroxyl groups, so that the polyol does not have to be distilled off and furthermore that the (trans)esterification reaction when performing the process in this way can be catalyzed by many Bronsted-Lowry acids in the absence of solvent or using a limited amount of solvent.

The present invention now provides an improved process to prepare esters of an amino carboxylic acid comprising the steps of reacting a polyol of the formula (I)

HO-A-OH (I)

where A is a carbon chain with 2 to 36 carbon atoms that can be aliphatic (linear or branched, saturated or unsaturated) or aromatic, or CH₂CH(OH)CH₂, CH₂C(CH₂OH)₂ CH₂, CH₂C(CH2OH)(CH₃)CH₂, CH₂C(CH₂OH)(CH₂CH₃)CH₂, p-tetrahydrofuran, erythritol or an ester of di- or tricarboxylic acids with ethylene or propylene glycol, and wherein A can optionally be alkoxylated with one or more alkylene oxide units or reacted with one or more hydroxy carboxylic acids,
with an aminocarboxylic acid of formula II or its cyclic amide of the formula III 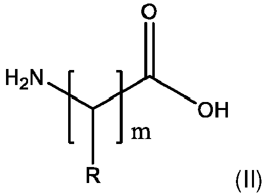 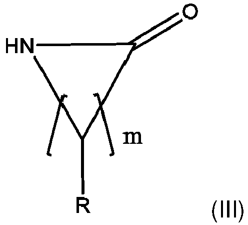 where m is an integer of 1-8 in formula II and 3-8 in formula III, each R independently is hydrogen or a C1-C4 alkyl group, or CH₂CH₂COOH, or CH₂COOH, or (CH₂)₄NH₂
in the presence of a Bronsted-Lowry acid at a temperature of between 60 and 200 degrees C wherein the total molar amount of aminocarboxylic acid or its cyclic amide to the molar amount of the polyol is between n:0.8 and n:1.5 wherein n is the total number of hydroxyl groups on the polyol and wherein the Bronsted-Lowry acid is not added to the reaction mixture until at least 50% of the total of the polyol and the aminocarboxylic acid or its cyclic amide are dosed.

The invention furthermore provides amine-functional esters of amino carboxylic acids as obtainable by the process of the invention.

The compounds obtainable by the process of the invention can in many embodiments be covered by the below structure 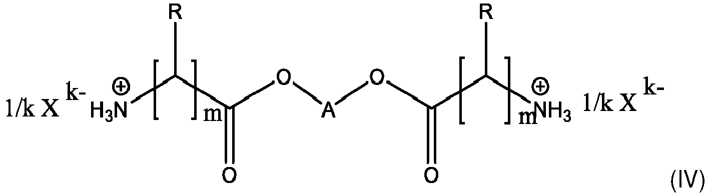 wherein k is a value of 1 to 3, each R independently is as defined above, A is as defined above, each m is as defined above, and X is an anion derivable from deprotonating a Bronsted-Lowry acid.

If following the above options for A, in the cases wherein the group A contains further hydroxyl substituents these may converted into groups wherein the proton of the OH group is substituted by the group of formula (V) 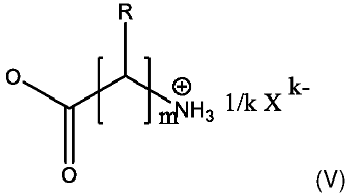 wherein k, each R independently, m and X are as defined above.

The compounds obtainable by the process of the invention may contain between 0 and 30 mole% on total moles of compounds (IV) of compounds below formula (VI) 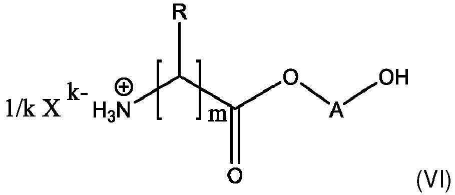 wherein k, each R independently, A, m and X are as defined above. Preferably the amount of compounds of formula VI is between 1 and 20 mole%, even more preferably between 2 and 15 mole%, most preferably 5 and 10 mole%, on total amount of compound IV.

The polyols that can be used in the process are compounds containing at least two hydroxyl groups of the formula HO-A-OH (I) where A is a carbon chain with 2 to 36 carbon atoms that can be aliphatic (linear or branched, saturated or unsaturated) or aromatic, or CH₂CH(OH)CH₂, CH₂C(CH₂OH)₂CH₂, CH₂C(CH₂OH)(CH₃)CH₂, CH₂C(CH₂ OH)(CH₂CH₃)CH₂, p-tetrahydrofuran, erythritol or esters of di(tri)carboxylic acids with ethylene or propylene glycol, optionally alkoxylated with one or more alkylene oxide unit or reacted with one or more hydroxy carboxylic acids.

Examples of polyols that are alkoxylated are HO-(CH₂-CH(R1)-O)p-CH₂-CH(R1)-(O-CH₂-CH(R1))p-OH, HO-(CH₂-CH(R1)-O)p-CH₂-CH=CH-CH₂-(O-CH₂-CH(R1))p-OH, HO-(CH₂-CH(R1)-O)p-CH₂-CH(CH₃)₂-CH₂-(O-CH₂-CH(R1))p-OH, HO-(CH₂-CH(R1)-O)ₚ-C₆H₄-(O-CH₂-CH(R1))p-OH, HO-(CH₂-CH(R1)-O)p-CH₂-C₆H₄-CH₂-(O-CH₂-CH(R1))p-OH, HO-(CH₂-CH(R1)-O)p-C₆H₁₀-(O-CH₂-CH(R1))p-OH, HO-(CH₂-CH(R1)-O)p-CH₂-C₆H₁₀-CH₂-(O-CH₂-CH(R1))p-OH, HO-(CH₂-CH(R1)-O)p-CH₂-(CH₂)n-CH₂-(O-CH₂-CH(R1))p-OH, HO-(CH₂-CH(R1)-O)p-CH₂-CH₂-CH₂-CH₂-(O-CH₂-CH₂-CH₂-CH₂)p-(O-CH₂-CH(R1))p-OH, HO-(CH₂-CH(R1)-O)p-(CH₂)i-CH((CH₂)jCH₃)-CH((CH₂)jCH₃)-(CH₂)i-(O-CH₂-CH(R1))p-OH, HO-(CH₂-CH(R1)-O)p-CH₂-(CH(O-CH₂-CH(R1))p-OH))d-CH₂-(O-CH₂-CH(R1))p-OH, HO-(CH₂-CH(R1)-O)p-CH₂-C(CH₂-(O-CH₂-CH(R1))p-OH)₂-CH₂-(O-CH₂-CH(R1))p-OH, HO-(CH₂-CH(R1)-O)p-CH₂-C(CH₂-(O-CH₂-CH(R1))p-OH)(CH₃)-CH₂-(O-CH₂-CH(R1))p-OH, HO-(CH₂-CH(R1)-O)p-CH₂-C(CH₂-(O-CH₂-CH(R1))p-OH)(CH₂CH₃)-CH₂-(O-CH₂-CH(R1))p-OH, wherein each p is independently an integer of from 0-25, as long as one p in a structure is at least 1, n is 1-10, i+j=16, d=1-4 and each R1 independently is hydrogen atom or C1-C2 alkyl group.

Examples of polyols that are alcohols reacted with hydroxy carboxylic acids are compounds of below formulae:
HO-(CH(Z)-C(O)-O)p-CH₂-CH(R1)-(O-C(O)-CH(Z))p-OH, HO-(CH(Z)-C(O)-O)p-CH₂-CH=CH-CH₂-(O-C(O)-CH(Z))p-OH, HO-(CH(Z)-C(O)-O)p-CH₂-CH(CH₃)₂-CH₂-(O-C(O)-CH(Z))p-OH, HO-(CH(Z)-C(O)-O)p-C₆H₄-(O-C(O)-CH(Z))p-OH, HO-(CH(Z)-C(O)-O)p-CH₂-C₆H₄-CH₂-(O-C(O)-CH(Z))p-OH, HO-(CH(Z)-C(O)-O)p-C₆H₁₀-(O-C(O)-CH(Z))p-OH, HO-(CH(Z)-C(O)-O)p-CH₂-C₆H₁₀-CH₂-(O-C(O)-CH(Z))p-OH, HO-(CH(Z)-C(O)-O)p-CH₂-(CH₂)n-CH₂-(O-C(O)-CH(Z))p-OH, HO-(CH(Z)-C(O)-O)p-CH₂-CH₂-CH₂-CH₂-(O-CH₂-CH₂-CH₂-CH₂)ₚ-(O-C(O)-CH(Z))p-OH, HO-(CH(Z)-C(O)-O)p-(CH₂)i-CH((CH₂)jCH₃)-CH((CH₂)jCH₃)-(CH₂)i-(O-C(O)-CH(Z))p-OH, HO-(CH(Z)-C(O)-O)p-CH₂-(CH(O-C(O)-CH(Z))p-OH))d-CH₂-(O-C(O)-CH(Z))p-OH, HO-(CH(Z)-C(O)-O)p-CH₂-C(CH₂-(O-C(O)-CH(Z))p-OH)₂-CH₂-(O-C(O)-CH(Z))p-OH, HO-(CH(Z)-C(O)-O)p-CH₂-C(CH₂-(O-C(O)-CH(Z))p-OH)(CH₃)-CH₂-(O-C(O)-CH(Z))p-OH, HO-(CH(Z)-C(O)-O)p-CH₂-C(CH₂-(O-C(O)-CH(Z))p-OH)(CH₂CH₃)-CH₂-(O-C(O)-CH(Z))p-OH, HO-(CH(Z)-C(O)-O)p-CH₂(CH₂)k-CH(R1)-(O-C(O)-CH(Z))p-OH, HO(CH(Z)-C(O)-O)p-CH₂-(CH(O-C(O)-CH(Z)-OH)p)e-CH2-(O-C(O)-CH(Z))pOH
wherein each p is independently an integer of from 0-25, as long as at least one p in a structure is at least 1, and each R1 independently is hydrogen atom or C1-C2 alkyl group, k is 0-10, if k>0 then R1 is hydrogen, Z is hydrogen or CH3, e=1-4.

Examples of polyols that are esters of di- or tricarboxylic acids with ethylene glycol or propylene glycol are compounds of below formula HO(CH₂CH(R1)-O)p-C(O)-CH₂-(C(OH)(C(O)(O-CH(R1)-CH₂)pOH)d-(CH₂)k-C(O)(O-CH(R1)-CH₂)pOH
wherein each p is independently an integer of from 0-25 as long as at least one p in a structure is at least 1, and each R1 independently is hydrogen atom or C1-C2 alkyl group, d=0-1, when d =1, then k=1; when d=0, then k=0-10

Most preferred polyols are selected from the group of ethylene glycol, propylene glycol, butylene glycol, polyethylene glycol, polypropylene glycol, polybutylene glycol, glycerol, poly tetrahydrofuran, pentaerythritol, trimethylolpropane, trimethylolethane, diethylenesuccinate, dimeric fatty alcohol, 1,6-hexandiol, 1,4-butandiol, 1,8-octandiol, 1,10-decandiol, erythritol, optionally alkoxylated or reacted with hydroxy carboxylic acid. When using polyethylene glycol, polypropylene glycol or polybutylene glycol these preferably contain 2 to 10 ethyleneoxide, propyleneoxide, resp butyleneoxide units, even more preferably 2 to 6 units.

Mixtures of two or more polyols may also be employed.

Preferred aminocarboxylic acids of formula II are glycine, alanine, valine, leucine, isoleucine, lysine, aspartic acid, and glutamic acid, more preferred are glycine or alanine. Further preferred aminocarboxylic acids of formula II are selected from the group of 6-aminohexanoic acid, 4-aminobutanoic acid.

In a preferred embodiment a cyclic amide of formula III is employed in the process of the invention. It is more preferred that in formula III m is 3, 4, 5 or 6. Most preferred are the cyclic amides of 6-aminohexanoic acid, 4-aminobutanoic acid.

It should be noted that a mixture of aminocarboxylic acids and/or of cyclic amides can also be employed in the process.

Preferred amine-functional esters of formula (IV) include

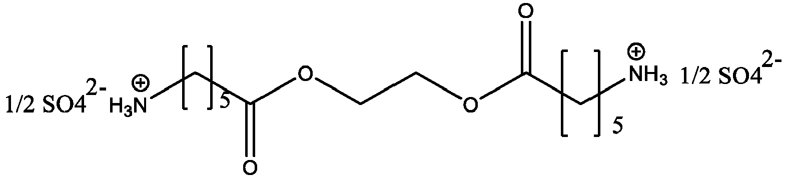

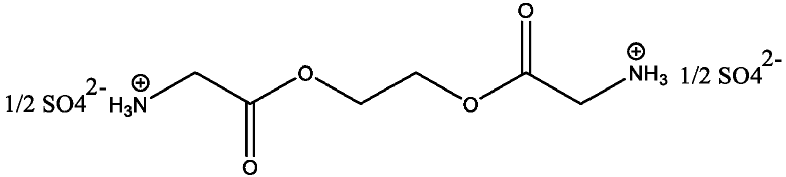

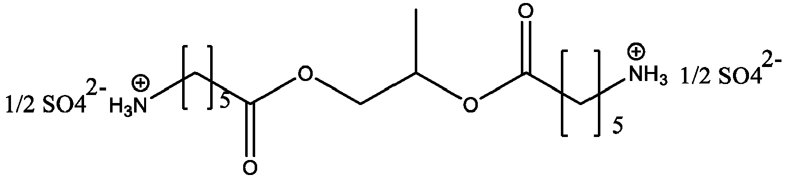

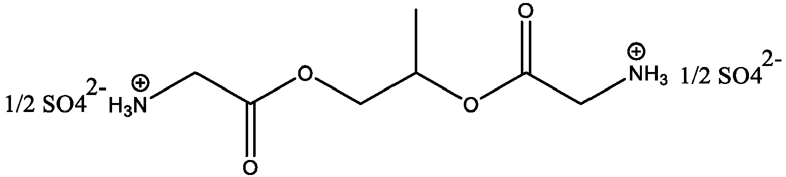

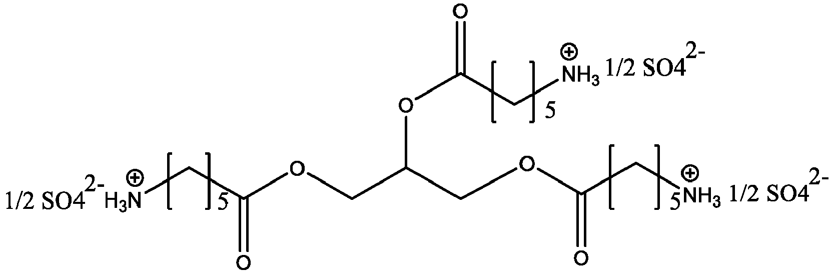

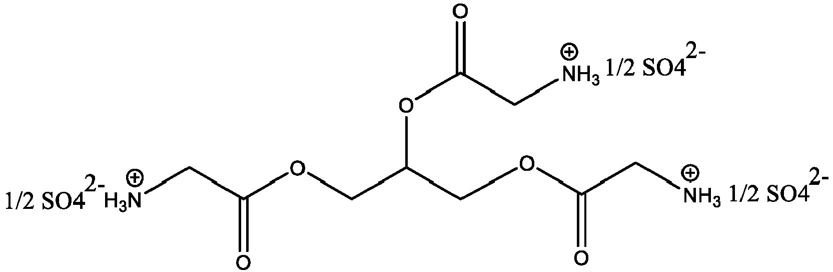

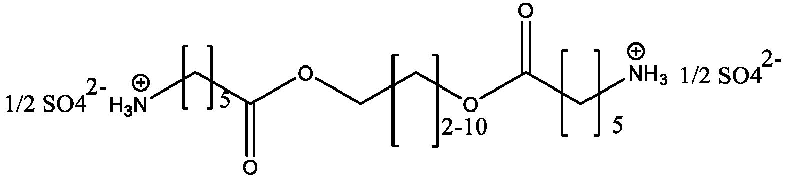

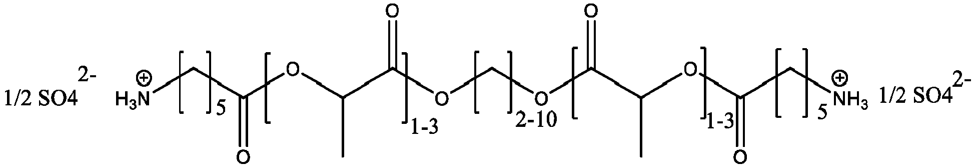

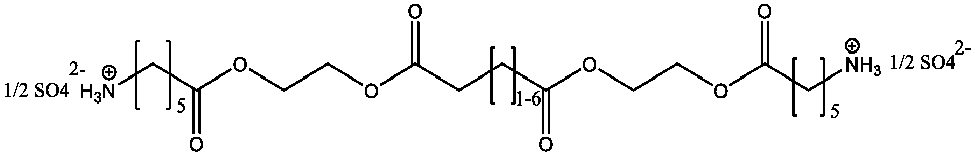

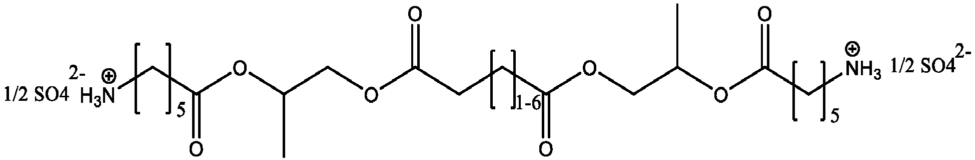

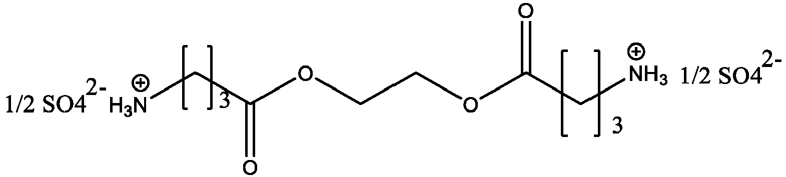

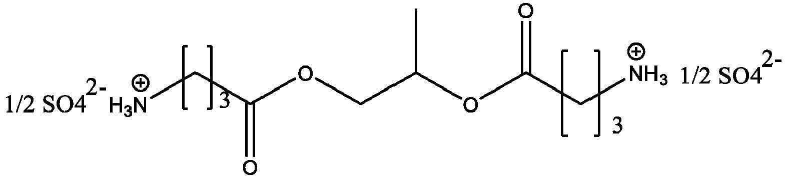

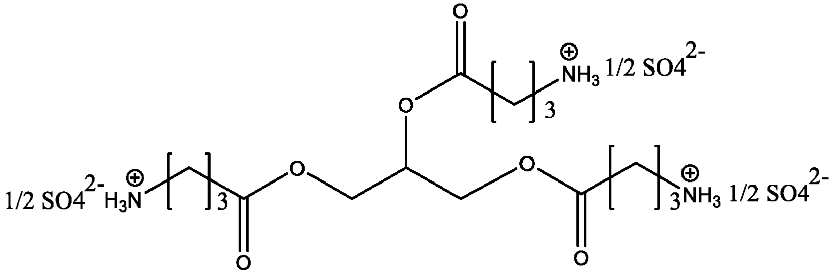

The compounds of formula (IV), (VI) were determined to have an improved biodegradability and/or chemical degradability compared to many commercially available amine-functional compounds that contain at least two amine groups, which adds to the environmental profile of any application in which they are used. They can be employed as alternatives with better degradability profile in applications wherein diamines/polyamines are presently used such as in the preparation of polymers that contain diamine monomers. Such polymers include polyurea, polyepoxide, and polyamide polymers and there is a desire to make such polymers biodegradable. Furthermore, they can be employed as building blocks wherein the amine is further modified to make imide- or (meth)acrylamide-functional monomers.
In preferred embodiments the compounds of formula (IV), (VI) are readily biodegradable.

In a preferred embodiment of the process of the invention the aminocarboxylic acid is used in its cyclic amide form (of formula III). The advantage of performing a transesterification reaction of a cyclic amide of an amino carboxylic acid instead of an esterification of an amino carboxylic acid or salt thereof is that it is much easier to control the amount of water in the process. When doing a transesterification reaction there is no net formation of water. When performing an esterification reaction, more water will form and such increased water content may give rise to side reactions, such as in particular under an acidic pH, a hydrolysis reaction wherein formed product falls apart in the starting compounds again. Also, when attempting to remove water from the reaction mixture, the reactants, most notably the polyols, might form an azeotrope with the water and get lost as well.

Furthermore, it has been found that the side reaction wherein the cyclic amide self-polymerizes that is suppressed in state of the art processes by diluting the reaction mixture with an excess of alkanol reactant was found to be similarly suppressed in the process of the invention, wherein the reactants cyclic amide and polyol are dosed such that around about 1 mole of cyclic amide is present per hydroxyl group on the polyol, especially in embodiments wherein the process is done under somewhat more moderate temperatures, such as temperatures under 150 deg C. Quite unexpectedly, in the process of the invention the (trans)esterification reaction takes similarly long as in the prior art processes wherein the alkanol is clearly overdosed and still very similar yields of desired product are obtained. Finally, it is an advantage that the excess of polyol does not need to be removed from the reaction mixture.

In the process of the invention the amino carboxylic acid or its cyclic amide derivative, and the polyol are preferably employed in a molar ratio of total aminocarboxylic acid and/or cyclic amide to polyol that is preferably between n:0.8 and n:1.3, more preferably between n:0.9 and n:1.1, wherein n stands for the number of hydroxyl groups on the polyol, most preferably both compounds are used in a substantially equimolar amount of about 1 mole of aminocarboxylic acid per hydroxyl group.

Unexpectedly the reaction can be performed under relatively mild conditions, such as a milder reaction temperature, and provide high yields of the ester if the polyol is used in the ratio of the present invention. Effectively the reaction was found to progress substantially towards the claimed ester product in which there is only a limited remaining amount of unreacted polyol and aminocarboxylic acid or cyclic amide and in which the inventors have only found trace amounts of only one side product that is the reaction product of polyol and Bronsted-Lowry acid.

In applications wherein a considerable amount of polyol or side products caused by not being able to control the amount of water, create a problem but a minor amount of unreacted polyol or aminocarboxylic acid or its cyclic amide are not an issue, the product of the process of the invention can be used without intermediate steps to remove impurities such as for example unreacted starting materials.

The Bronsted-Lowry acid is preferably not added to the reaction mixture until at least 50% of the total of the polyol and at least 50% of the total of the (cyclic amide of the) aminocarboxylic acid are added. In a preferred embodiment at least 75% of the total of the polyol and at least 75% of the aminocarboxylic acid or its cyclic amide are dosed before the Bronsted-Lowry acid is added to the reaction mixture, even more preferably at least 90%. In a preferred embodiment, at least part of the polyol is first dosed to the reactor and next at least part of the aminocarboxylic acid or its cyclic amide, after which the Bronsted-Lowry acid is added. It however is also possible to dose the aminocarboxylic acid or its cyclic amide partially or completely before the polyol is dosed to the reactor or to simultaneously dose the cyclic amide and the polyol after which the Bronsted-Lowry acid is added.

The temperature is preferably increased to at least 60 deg C and to up to 200 deg C after the reactants and the Bronsted-Lowry acid have been dosed. It is beneficial to first increase the temperature to between between 60 and 100 deg C and next to a temperature between 100 and 150 deg C, or even more preferably 110 and 140 deg C.

The process can be performed in a solvent or without a solvent. If a solvent is used it is preferably an organic solvent that is not an aromatic solvent, not a halogenated alkane solvent and not an alcoholic solvent. Solvents that are suitable to use in the process of the invention can be selected from the group of alkane and ether solvents with a boiling point higher than 80 deg C more preferably higher than 100 deg C, even more preferably higher than 120 deg C. Water can also be used. The process is preferably performed with an amount of solvent that is 0 to 50 wt%, preferably 0 to 25 wt%, most preferably 0-10 wt%, based on total reactants.

In a preferred embodiment during the process the molar amount of water on total moles of polyol is between 0 and 10 mole%, more preferably 0.01 and 5 mole%, most preferably 0.1 and 2 mole%.

After the reaction is completed, optionally (to decrease viscosity) the product can be diluted with water and/or an organic polar solvent. The preferred organic solvents are glycolic solvents, such as propylene glycol, triethylene glycol, ethylene glycol, 2-methoxyethanol, glycerol, or isopropanol.

The Bronsted-Lowry acid is preferably used as a concentrated (i.e. 20 to 100 wt%) aqueous solution and dosed to the reaction mixture containing all the aminocarboxylic acid or its cyclic amide and the polyol in portions to control the exothermal effect of the reaction.

The Bronsted-Lowry acid is not an aminocarboxylic acid. The Bronsted-Lowry acid is preferably an acid with a pKa of between -10 and 3. The Bronsted-Lowry acid is even more preferably an inorganic acid, even more preferably it is sulfuric acid, phosphoric acid or a hydrohalic acid and most preferably it is sulfuric acid as hydrohalic acids such as HCI in embodiments result in solid products and phosphoric acid results in lower conversions. Moreover, both HCI and H3PO4 as a liquid are available as solutions with quite some water (HCI 37% conc, H3PO4 85% conc) while H2SO4 is available in substantially water-free form (such as 95-98% concentrate). The use of H2SO4 therefore allows to get products of high conversion (>90%) in a liquid form, and moreover no need to evaporate water exists.

X is in a preferred embodiment an anion derived from an inorganic Bronsted-Lowry acid, more preferred a halogenide, sulphate, hydrogen sulfate, hydrogen phosphate, dihydrogen phosphate, or phosphate anion. Most preferably, X is a sulfate or hydrogen sulfate anion.

The process can be done under reduced pressure but is preferably performed at a pressure that is atmospheric.

The product can optionally be neutralized by a base to a pH from 3-7. The product can be purified by methods available to someone skilled in the art. However, because of the low level of side products, it can also be used without further processing or purification steps, such as as a surfactant.

The process is very favorable for polyols that have a boiling point up to 220 deg C, or, for polyols that can form an azeotrope with water having a boiling point lower than 220 deg C. Compared to the state of the art processes, in the process of the invention when using such polyols, they will not be stripped from the reaction mixture when reaction water is removed, as in the process of the invention no such water is formed and hence does not need to be removed. Many polyols that can be used in the process of the invention have a boiling point in this range (especially the polyols that are smaller or that contain some branching in their structure).

The process is preferably done at a temperature of between 60 and 150 deg C, more preferably between 80 and 145 deg C, most preferably 110 and 140 deg C.

### Examples

### Compounds used

ε-Caprolactam (ex Acros Organics)
pyrrolidone (ex Acros Organics)
Monoethylene glycol (ex Sigma-Aldrich)
Polytetrahydrofurane (ex Sigma-Aldrich)
triethyleneglycol (ex Sigma-Aldrich)
glycerol (ex Sigma-Aldrich)
H2SO4 (95%) (ex VWR)

### Example 1 - Preparation of ethane-1,2-divl bis(6-aminohexanoate) sulphate salt

A 250 mL flanged glass reactor with overhead stirrer, thermometer and cooler was loaded with ε-caprolactam (89,9 g, 0.79 mol) and ethylene glycol (24,6 g, 0.4 mol). The mixture was heated to 80 deg C. Then, sulphuric acid (95% in water 55,94 g, 0.54 mol) was added and the mixture was heated to 140 deg C. After 8 hours, the mixture was cooled to room temperature and analysed by NMR. The product was obtained as a viscous liquid. The product contained ethane-1,2-diyl bis(6-aminohexanoate) salt in a high yield of more than 80% and a small amount of hydroxyethyl-6-aminohexanoate sulphate salt.

### Example 2 - Preparation of diesterdiamine from triethyleneglycol and caprolactam

Caprolactam (90.2 g, 797 mmol) and triethylene glycol (59.8 g, 399 mmol) were charged in a 500 mL flanged glass reactor and heated to 60 °C (externally measured temperature) using mechanical stirring, under a stream of nitrogen. Sulfuric acid (60 g, 578 g) was added over 25 mins and the temperature was then increased to 135 °C and the reaction was stopped after approximately 13 h. The product was isolated and analyzed by ¹H NMR.

### Example 3 - Preparation of diesterdiamine from poly-THF and caprolactam

Caprolactam (16.9 g, 149 mmol) and Terathane 1400 (102.6 g, 75 mmol) were charged in a 1L flanged glass reactor and heated to 60 °C (externally measured temperature) using mechanical stirring, under a stream of nitrogen. Sulfuric acid (11 g, 108 mmol) was added over 25 mins and the temperature was then increased to 135 °C and the mixture was further stirred for approximately 19 h. The product was isolated and analyzed by ¹H NMR. Approximately 70% yield was established.

### Example 4 - Preparation of triestertriamine from glycerol and caprolactam

Caprolactam (212 g, 1880 mmol) and glycerol (57.6 g, 625 mmol) were charged in a 500 mL flanged glass reactor and heated to 60 °C (externally measured) using mechanical stirring, under a stream of nitrogen. Sulfuric acid (118 g, 1100 mmol) was added over 45 mins and the temperature was then increased to 135 °C and the mixture was further stirred for approximately 14 h. The product was isolated and analyzed by ¹H NMR. The product contained propane-1,2,3-triyl tris(6-aminohexanoate) salt in a high yield of more than 80%.

### Example 5 - Preparation of diesterdiamine from ethylene glycol and 2-pyrrolidinone

2-pyrrolidinone (93.7 g, 1100 mmol) and ethylene glycol (34.5 g, 556 mmol) were charged in a 1L flanged glass reactor and heated to 60 °C (externally measured temperature) using mechanical stirring, under a stream of nitrogen. Sulfuric acid (82 g, 790 mmol) was added over 45 mins and the temperature was then increased to 135 °C and the mixture was further stirred for approximately 25 h. The product was isolated and analyzed by ¹H NMR. Approximately 80% yield was established.

## Claims

1. Process to prepare amine-functional esters of an amino carboxylic acid comprising the steps of reacting a polyol of the formula (I)
HO-A-OH (I)
where A is a carbon chain with 2 to 36 carbon atoms that can be aliphatic (linear or branched, saturated or unsaturated) or aromatic, or CH₂CH(OH)CH₂, CH₂C(CH₂ OH)₂CH₂, CH₂C(CH₂OH)(CH₃)CH₂, CH₂C(CH₂OH)(CH₂CH₃)CH₂, p-tetrahydrofuran, erythritol or an ester of di- or tricarboxylic acids with ethylene or propylene glycol, and wherein A can optionally be alkoxylated or reacted with hydroxy carboxylic acids,
with an aminocarboxylic acid of formula II or its cyclic amide of the formula III 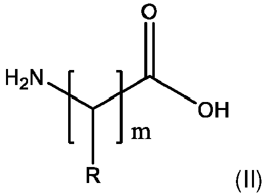 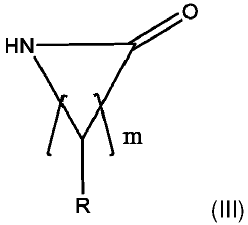 where m is an integer of 1-8 in formula II and 3-8 in formula III, each R independently is hydrogen or a C1-C4 alkyl group, or CH₂CH₂COOH, or CH₂COOH, or (CH₂)₄NH₂
in the presence of a Bronsted-Lowry acid at a temperature of between 60 and 200 degrees C wherein the total molar amount of aminocarboxylic acid or its cyclic amide to the molar amount of the polyol is between n:0.8 and n:1.5 wherein n is the total number of hydroxyl groups on the polyol and wherein the Bronsted-Lowry acid is not added to the reaction mixture until at least 50% of the total of the polyol and the aminocarboxylic acid or its cyclic amide are dosed.

2. Process of claim 1 wherein the total molar amount of aminocarboxylic acid or cyclic amide to the molar amount of the polyol is between n:0.9 and n:1.1.

3. Process of claim 1 or 2 wherein a cyclic amide of formula III is used wherein m is 3, 4, 5 or 6.

4. Process of any one of claims 1 to 3 wherein the Bronsted-Lowry acid is sulfuric acid or phosphoric acid, preferably sulfuric acid.

5. Process of any one of claims 1 to 4 wherein the polyol is selected from the group of ethylene glycol, propylene glycol, butylene glycol, polyethylene glycol, polypropylene glycol, polybutylene glycol, glycerol, poly tetrahydrofuran, pentaerythritol, trimethylolpropane, trimethylolethane, diethylenesuccinate, dimeric fatty alcohol, 1,6-hexandiol, 1,4-butandiol, 1,8-octandiol, 1,10-decandiol, erythritol, optionally alkoxylated or reacted with hydroxy carboxylic acid.

6. Process of any one of claims 1 to 5 wherein the polyol has a boiling point of up to 220 deg C, or, can form an azeotrope with water having a boiling point up to 220 deg C.

7. Process of any one of claims 1 to 6 wherein the temperature is between 60 and 150 deg C.

8. Process of any one of claims 1 to 7 wherein the process is done without solvent or with solvent in an amount of solvent of 0 to 50 wt%, preferably 0 to 25 wt%, most preferably 0-10 wt%, based on total reactants.

9. Process of claim 8 wherein the solvent is an organic solvent that is not aromatic and does not contain halogens or alcohol units.

10. Amine-functional esters obtainable from the process of any one of preceding claims 1 to 9.

11. Amine-functional esters of claim 10 having a structure of formula (IV) 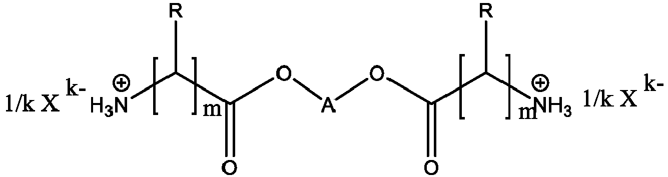 wherein k is a value of 1 to 3, each R independently is as in claim 1, A is as in claim 1, each m is as in claim 1, and X is an anion derivable from deprotonating the Bronsted-Lowry acid.

12. Amine-functional esters of claim 10 or 11 wherein X is sulfate or hydrogen sulfate.

13. Amine-functional esters of any one of claims 10 to 12 containing between 0 and 30 mole% on the basis of total moles of compounds (IV) of compounds of formula (VI) 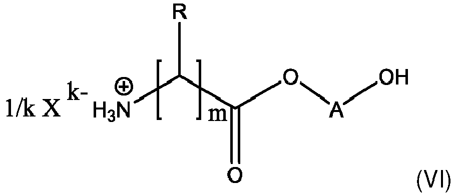 wherein k, each R independently, A, m and X are as in claim 11.

14. Use of amine-functional esters of any one of claims 10 to 13 as amine-functional monomers in the preparation of polymers, preferably of polyurea, polyepoxide or polyamide polymers or as building blocks to make imide or (meth)acrylamide monomers.

15. Polymers, preferably polyurea, polyepoxide, or polyamide, containing amine-functional esters of any one of claims 10 to 13 as a monomer.
